# EUROPEAN PATENT APPLICATION

(11) **EP 3 583 929 A1**
(43) Date of publication of application: **25.12.2019**
(21) Application number: 19183812.7
(22) Date of filing: 15.12.2014
(51) Int. Cl.: A61F 13/15, B29C 65/08, B29C 65/00, B29L 31/48

(54) **A METHOD AND APPARATUS FOR THE TRANSVERSE WELDING OF OVERLAPPING ELASTIC STRUCTURES**

(30) Priority: 17.12.2013 IT TO20131031
(62) Divisional of application: 14197860.1
(71) Applicant: Fameccanica.Data S.p.A., 65129 Pescara (IT)
(72) Inventor: SABLONE, Gabriele, I-65125 Pescara (IT)
(74) Representative: Marchitelli, Mauro

(57) **Abstract**

A method for producing sanitary articles of the pre-fastened type in the form of pants (10, 10') that involves the transverse welding of a composite tape (100, 100') formed of at least two overlapping elastic structures (1a, 1a', 1b, 1b'), which continuously advance in a longitudinal direction (MD) at a first feeding speed (V1) and at a first feed tension (T1);
- slowing down (70) said composite tape (100, 100'), taking it to a second speed (V2) and a second tension (T2), respectively less than said first feeding speed (V1) and said corresponding first feed tension (T1),
- feeding said composite tape (100, 100') at said second speed (V2) and tension (T2) to a welding station (80) and welding (80) said composite tape (100, 100') with welding lines (13) transverse to said longitudinal feed direction (MD), and
- feeding said composite tape (100, 100') at said second speed (V2) and tension (T2) to a cutting station (90) and cutting (90) said composite tape (100, 100') with cuts (12) transverse to said longitudinal feed direction (MD).

## Description

### Technical field

The present description refers to the transverse welding of a composite tape formed from two or more layers of overlapping elastic material, which can be used for producing sanitary articles such as training-pant products of the pre-fastened type.

The reference to this specific field of application should not be, however, interpreted in a limiting sense of the scope of the invention.

### Technological background

A common technique for the production of sanitary products wearable in the manner of pants, of the pre-fastened type normally referred to as "training pants" or "pull-ups" consists of producing a composite tape formed of a continuous chain of product blanks, aligned in order to have the transverse axis of each single absorbent article coincident with the direction of longitudinal advancing of the tape itself.

The continuous composite tape is folded in two around a longitudinal axis coaxial with said transverse axis of each single absorbent article, so that the longitudinal end portions of the composite tape overlap with each other.

The two edges of the composite tape overlapping in this way are connected together with transverse weld lines, so as to define the waist and leg openings of each absorbent article. The continuous chain of blanks is then subjected to a cutting operation, at the welds, to divide the finished products from each other.

This particular manufacturing methodology is normally identified by the term "Cross-Direction" (or CD), distinguishing it from the more traditional form of production, said "Machine Direction" (or MD), which forms the absorbent articles while they travel with their longitudinal axis parallel to the direction of production.

An example of a method for manufacturing absorbent sanitary products of the CD type is described in the documents EP-A-1 013 251, IT-0001379452, TO2012A000209 and TO2012A000210 by the same applicant.

In the "Cross Direction" methods, it is necessary to ensure a correct positioning of the transverse welds of each product, in order to correctly define the waist and leg openings of each single diaper. Furthermore, it is important that the cutting line intended to divide the individual products from each other is correctly positioned at the welds produced between two adjacent absorbent products and, very importantly, both the welds and the cuts should present a constant angular position with respect to the advancing direction of the sheet, or, in other words, they must always present, at least the welds, the same angular relation with respect to the edges of the composite tape.

As well known to the skilled person, producing the transverse welding lines on the composite tape currently constitutes one of the major technological limitations to the operation speed of the production lines.

Indeed, the welding system must guarantee an adequate sealing force of the transverse welds that produce the closure of the diaper around the user's waist.

In order to guarantee a resistance that allows the safe use of the absorbent article, the side welds must typically present values of tensile strength of the order of 1000 grams per inch (2.54 cm).

The apparatuses that are used for producing transverse welds are, typically, welding devices that use ultrasonic equipment that provides the energy to carry out the welding of the composite tape with a forging-type process. In practice, the welding is carried out by repeatedly striking, with a strong intensity, the layers of material interposed between the vibrating device of the ultrasonic system and the respective counter-roller, which is provided with a series of protuberances (or welding pattern), where the material of the tape is repeatedly pressed by the blows inflicted by the vibrating element of the ultrasonic system.

Due to the repeated blows, the material of the tape interposed between the welding pattern and the vibrating element of the ultrasonic system decreases in thickness by compressing and moving toward the side edges of the protuberances of the counter-roller, generating the heat necessary to melt all the layers of material.

In order to obtain an acceptable weld, it is necessary to produce a minimum number of blows that, for each composite tape, may vary according to the type of material, the number of layers and the configuration of the welding pattern. Furthermore, increasing the speed with which the tape passes through the welding system decreases the number of blows that can be inflicted to the tape by an ultrasonic vibrating system at a given frequency, thereby negatively affecting the quality of the weld.

### Object and summary

The object of the present invention is to provide a method and an apparatus for producing transverse weld lines on a composite tape, which can be used for producing sanitary articles wearable in the manner of pants of the pre-fastened type, which is also successfully usable in production lines operating at high production speeds.

According to the present invention, this object is achieved by a method forming the subject of claim 1 and by an apparatus according to claim 9.

The claims form an integral part of the disclosure provided in relation to the invention.

### Brief description of the drawings

The invention will now be described, purely by way of non-limiting example, with reference to the accompanying figures, in which:
- Figure 1 is a perspective view of an absorbent product of the pre-fastened pant type producible with the apparatus according to a preferred embodiment of the present invention, in the closed configuration and ready for use,
- Figure 2 is a schematic perspective view of the absorbent product of Figure 1 in the open extended configuration,
- Figures 3a and 3b represent the composite tapes formed of the semi-finished blanks of two types of absorbent products of the pre-fastened pant type producible with the apparatus according to a preferred embodiment of the present invention,
- Figure 4 is a schematic perspective view of the production steps of an absorbent article according to a preferred embodiment of the present invention,
- Figure 5 is a schematic view of a braking device of the present invention according to a preferred embodiment,
- Figure 6 is a schematic perspective view of the production steps of an absorbent article according to a further preferred embodiment of the present invention, and
- Figure 7 is a schematic view of a braking device of the present invention according to a further preferred embodiment.

### Detailed description of the embodiments

Below, a manufacturing method of an absorbent article 10 will be described according to a preferred embodiment of the present invention.

In the following description, various specific details are illustrated aimed at a thorough understanding of the embodiments. The embodiments can be implemented without one or more of the specific details, or with other methods, components, materials, etc. In other cases, known structures, materials, or operations are not shown or described in detail to avoid obscuring various aspects of the embodiments.

The reference to "an embodiment" in the context of this description indicates that a particular configuration, structure or characteristic described in relation to the embodiment is included in at least one embodiment. Therefore, phrases such as "in an embodiment", possibly present in different places of this description do not necessarily refer to the same embodiment. Furthermore, particular conformations, structures, or characteristics can be combined in any suitable manner in one or more embodiments.

The references used herein are for convenience only and therefore do not define the field of protection or the scope of the embodiments, therefore, the definitions "front" or "rear" referring to the absorbent sanitary article only depend on the end use of the product itself, or rather as it is worn by the user, while the definitions "upper" or "lower" relative to any elements present in one or more embodiments described only depend on the point of view of the observer, with respect to the drawings attached to this document without, therefore, limiting the principle and/or the scope of the invention.

Note that in the following description of the drawings, elements similar to each other will be designated with the same reference numeral.

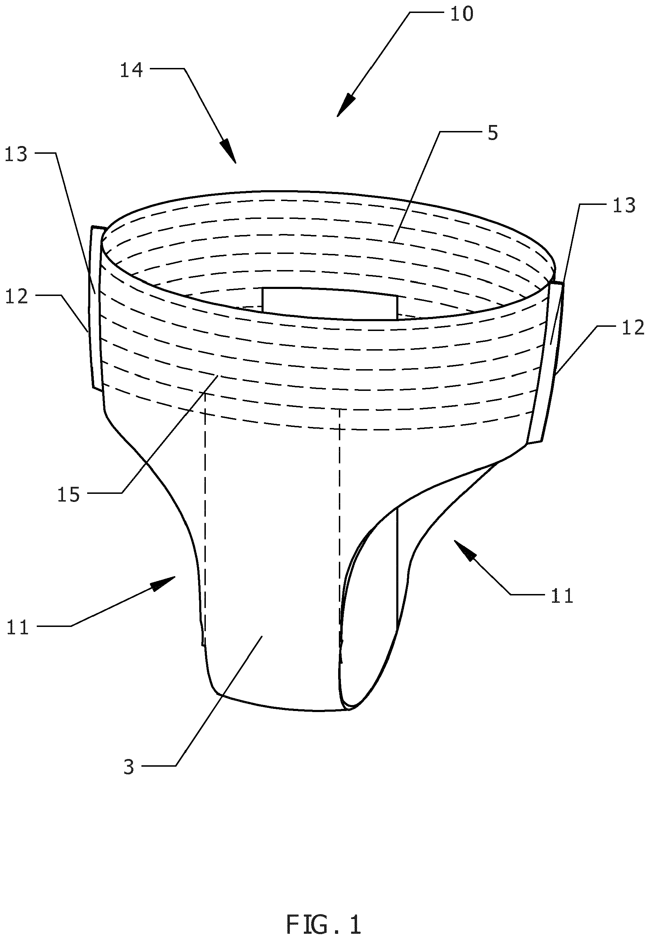

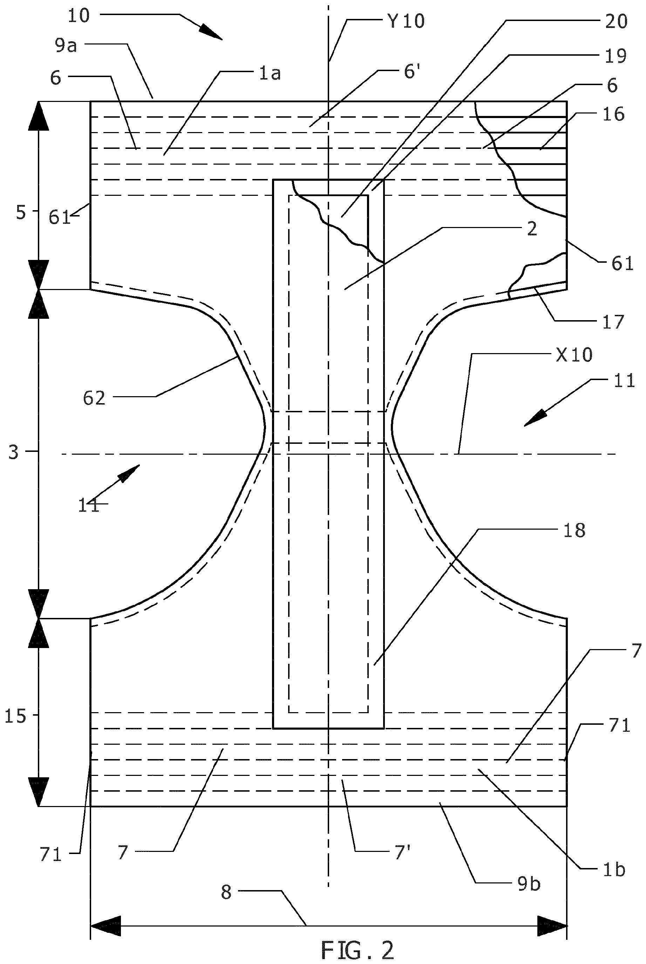

In Figures 1 and 2, a sanitary article 10 is represented, which can be produced with a method and an apparatus according to the disclosures provided by the present invention.

In Figure 1, the sanitary article 10 is represented in its closed condition, where it presents a conformation essentially comparable to pants. Instead, Figure 2 shows the same product 10 in the open and extended configuration, in which it is possible to identify a first waist region 5, a second waist region 15 and a crotch region 3 located between the two waist regions 5 and 15, as well as a pair of mutually perpendicular axes, longitudinal Y10 and transversal X10.

In the first waist region 5, it is possible to identify a pair of first side panels 6 separated by a first central waist region 6' interposed between them. Similarly, in the second waist region 15, a second central waist region 7' can be identified that separates the second waist panels 7. On each of the side panels it is possible to identify a distal edge 61 for the first side panels 6 and 71 for the second side panels 7.

The absorbent article 10 is folded around its transverse axis X10 to assume its pant-configuration so that the two waist regions overlap with each other in order to be able to carry out the transverse welds 13 that join together the distal edges 61, 71 of the side panels 6, 7 of the diaper and that confer the characteristic pant-conformation to it, defining the opening 14 for the waist and the two openings 11 for the legs, as shown in Figure 1.

The first waist region 5 and the second waist region 15 are typically elasticated, for example with elastic elements 16 in the form of threads. The two waist regions 5, 15 are interconnected by the crotch region 3 in which, typically, an absorbent insert 2 is present.

The first and the second waist regions 5, 15 are elasticated as they are used to maintain the absorbent insert 2 adherent to the user's body in a comfortable manner, even when it becomes heavy after having absorbed body fluids.

Diapers of this type can have different configurations from each other, both regarding the absorbent insert 2, and regarding the first and second waist regions 5 and 15.

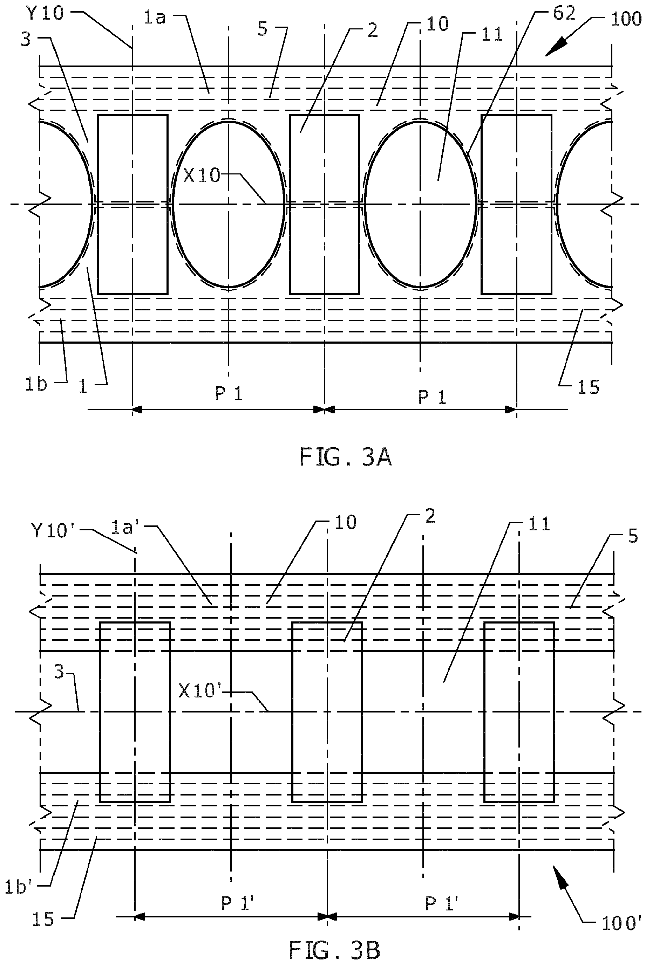

In Figures 3a and 3b, two embodiments are represented for producing sanitary articles 10 and 10', by way of example, well known to the skilled person. The continuous tapes formed of semi-finished blanks illustrated in Figures 3a and 3b have different structures from each other, but both can be obtained using the method of the present invention.

In Figure 3a, the absorbent article 10 presents the first waist region 5, the second waist region 15 and the crotch region 3, produced with a single tape 1, on which, typically, a series of cuts 62 are made, of appropriate shape, each of which contributes to define the contour of the leg openings 11 of two consecutive sanitary articles 10. The absorbent insert 2 is typically placed between two consecutive cuts 62.

In the absorbent article 10' obtainable by the chain of blanks of Figure 3b, the first waist region 5 and the second waist region 15 are formed of two tapes 1a' and 1b' separate and parallel to each other, while the absorbent insert 2 joins the two tapes 1a' and 1b' to each other, and forms the crotch region 3 and the opening for the legs 11.

For both of the absorbent articles 10 and 10', the absorbent insert 2 presents a structure in which the following features are recognizable (in addition to various other accessory elements), as illustrated in Figure 2, which represents, in particular, an article 10 obtainable by the chain of blanks of Figure 3a:
- an upper layer or topsheet 18 permeable to evacuated body fluids, intended to be facing the user's body;
- a lower layer or backsheet 19 impermeable to body fluids; and
- an absorbent core 20 interposed between the topsheet 18 and the backsheet 19 for absorbing and retaining the exudates.

On the absorbent product 10, and in particular on the absorbent insert 2, other elements may be present that contribute to increase the characteristics of wearability and absorbency, all well known to persons skilled in the art and of which extensive documentation is available.

The absorbent insert 2 and the tape 1, as illustrated in Figure 3a, can be made integral with each other by means of strips of adhesive and/or by means of welds produced with any system known per se, such as for example thermo-mechanical or ultrasonic welding.

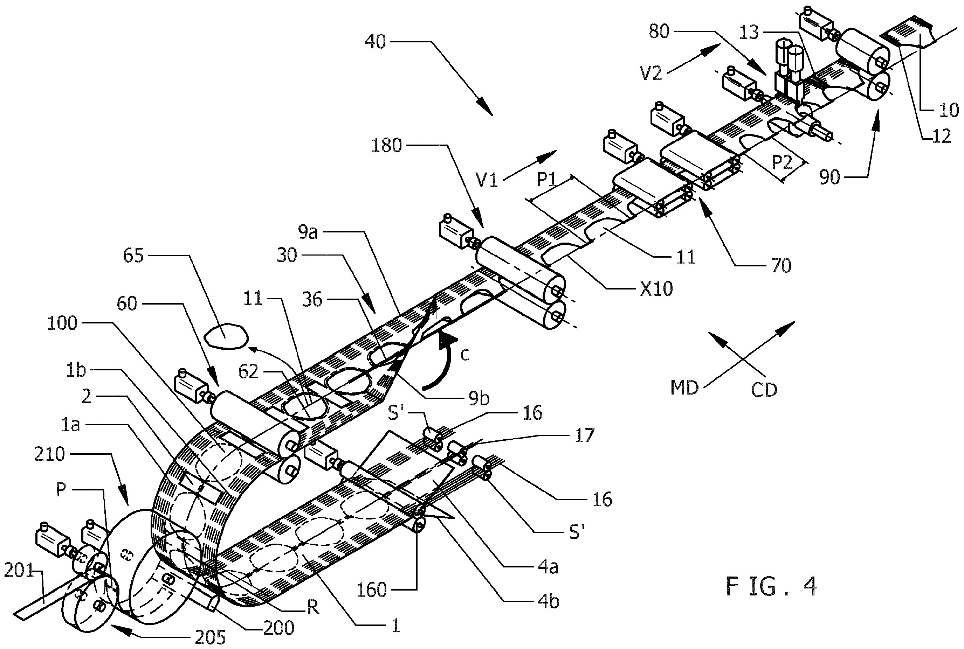

As already mentioned, the two types of sanitary articles 10 and 10', although having structural differences, can both be produced by the manufacturing method 40 according to a first preferred embodiment illustrated in Figure 4.

Below, therefore, apart from specific references, the method 40 will be described of the embodiment illustrated in Figure 4 with reference to the sanitary article 10 illustrated in Figures 1, 2 and 3a.

In Figure 4, a manufacturing method 40 is schematically shown according to a preferred embodiment of the invention, in which the production steps of the composite tape of elastic material 1 and of the relative sanitary articles 10 are represented.

In the manufacturing method 40 according to the preferred embodiment illustrated in Figure 4, it is possible to identify two main directions MD and CD perpendicular to each other, wherein the first direction MD is parallel to the production line flow.

In the preferred embodiment of Figure 4, the manufacturing method 40 of the absorbent article 10, includes a first step in which the tape 1 is produced.

A first and a second sheet 4a, 4b and the appropriately tensioned elastic elements 16 are joined together in a fixing station 160. The first sheet 4a and the second sheet 4b, in combination with the elastic elements 16, form a first elastic structure 1a and a second elastic structure 1b at the longitudinal end portions of the tape 1, each of which contributes to form, respectively, the first waist region 5 and the second waist region 15 of the absorbent article 10.

The inner sheet 4a and the outer sheet 4b are each supplied to the fixing station 160 by a respective unwinding source, not shown in Figure 4 but of a type known in the art, such as, for example, limiting ourselves to patent documents of the applicant, EP 1 013 585 A1.

The elastic elements 16 can be also conveyed to the fixing station 160 from a feed system S', of known type, such as, for example, the unwinder for elastic materials on a reel described in US 6676054.

Typically, both the inner sheet 4a and the outer sheet 4b are supplied to the welding station 160 at a first speed V1.

As is well known to the skilled person, the first speed V1 is equal to the transverse width 8 of the absorbent article 10 in an open and extended condition (as represented in Figure 2 or in Figures 3a and 3b) multiplied by the number N of absorbent articles that the production line is able to produce in the unit of time (1 second).

The elastic threads 16 used to produce the elastic structures 1a and 1b (forming the first and the second waist regions 5, 15 of the absorbent article 10) are fed to the fixing station 160 with a speed V16, less than said speed V1.

Normally, the two speeds V1 and V16 are very different from each other. Typically, the feeding speed V16 of the elastic elements 16 is about half of the first feeding speed V1 of the sheets 4a and 4b. The difference in speed between the sheets 4a and 4b and the elastic threads 16 causes an elongation of the elastic threads 16 proportional to the difference in speed. In this way, the required characteristics of elasticity and tension are conferred to the elastic structures 1a and 1b. The first elastic structure 1a and the second elastic structure 1b of the tape 1 (produced by lamination of the inner sheet 4a, the outer sheet 4b and the elastic elements 16) present a state of tension T1 proportional to the difference in speed (V1, V16) of their component elements.

Apparatuses and methods suitable for performing the functions of the fixing unit 160 are well known in the art and may use various technologies using adhesive or welding systems that can be implemented with heated rollers or with ultrasound apparatuses. Limiting ourselves to technologies that use application systems of hot-melt adhesives, the latter can be applied on one or both sheets or directly onto the elastic elements, or, alternatively, both on the sheets and on the elastic elements.

The welding unit 160 is typically provided with a pair of counter-rotating motorized rollers that press against each other, in order to adhere together all the materials passing through it and that make up the elasticated structures 1a and 1b. Typically, each of said rollers can be provided with a non-stick treatment to reduce contamination from the adhesive, if used in the manufacturing process.

To release the state of tension T1 of the elastic structures 1a and 1b, as well known to the skilled person, the elastic elements 16 must return to their resting condition, or rather, must return to a length corresponding to the feeding speed V16, which is typically half of the feeding speed V1 of the two sheets 4a and 4b. In other words, to cancel the tension T1, the elastic elements 16 must retract and return to their original length, approximately equal to half of the transverse width 8 of the absorbent article 10 in an open and extended condition. In this way, the elastic structures 1a and 1b retract, forming the characteristic "pleating" due precisely to the fact that said elastic elements 16 and said sheets 4a and 4b are joined together.

The fixing unit 160 is also able to perform the function of the control station of the feeding speed of the tape 1 (and therefore of the elastic structures 1a and 1b) toward all other processing stations downstream of it.

As illustrated in Figure 4, in the preferred embodiment, during the lamination step of the inner sheet 4a and the outer sheet 4b with the elastic elements 16, it is possible to join further elastic elements 17 to said sheets, suitable for elasticizing the leg openings 11 so as to confer greater wearability to the absorbent article 10.

Methods and apparatuses for the application of elastic elements suitable for improving the wearability of the absorbent article 10 conferring an elastic structure at the opening of the legs 11 are well known in the art. An example of equipment and method suitable for carrying out an application of elastic elements of this type is described in EP 1 842 516 A1 by the same Applicant.

In the preferred embodiment illustrated in Figures 1, 2 and 3a, the two sheets 4a, 4b can be of nonwoven fabric, which may have undergone treatments that render them water-repellent, so as to contain any loss of liquids and avoid any possible contamination of the user's clothing with which the sanitary article 10 may come into contact.

The elastic elements 16 and 17 can be selected from several materials available on the market, such as strips of synthetic rubber produced by Fulflex or Lycra® threads produced by Invista. Otherwise, on the market there is a wide range of natural or synthetic materials, able to be conveniently used to produce an absorbent article 10.

In the preferred embodiment of Figure 4, after having produced the tape 1, i.e. the elastic structures 1a and 1b, the device 20 applies absorbent inserts 2 onto it in order to produce a composite tape 100 formed of a continuous chain of product blanks aligned so as to have the transverse axis X10 of each absorbent article 10 parallel to the MD direction of the production line flow.

The absorbent inserts 2 are typically produced according to a method called "Machine Direction", i.e. with the longitudinal axis of the insert parallel to the MD direction of the production line flow.

The tape 201 of blanks of absorbent inserts 2 is typically fed to a cutting unit 205, which segments it so as to produce the individual absorbent inserts 2, and then to a Turn and Repitch device 210 which picks up each of said absorbent inserts 2 directly from the cutting unit 205 at a gripping point P and changes their orientation by rotating them by 90° in order to make the transversal axis X2 parallel to the production direction MD of the machine.

The Turn and Repitch device 210, prior to depositing the absorbent insert 2 on the tape 1 at the release point R, between the first elastic structure 1a and the second elastic structure 1b, synchronizes the speed of each absorbent insert 2, with the speed V1 of the tape 1.

Before the release point R, a device for applying adhesive 200 is typically inserted on the tape 1, which spreads a film of glue, of suitable size and amount, in order to join the tape 1 together, and in particular the first elastic structure 1a and the second elastic structure 1b with the absorbent inserts 2.

In this way, a composite tape 100 is produced, formed of a tape 1, which comprises a first elastic structure 1a, and a second elastic structure 1b on which a sequence of absorbent inserts 2 is applied, spaced apart at a first constant pitch P1, and with the respective transverse axis X2 coinciding with the transverse axis X10 of the corresponding absorbent article 10 and parallel to the MD direction of the production line flow.

An example of a Turn and Repitch device 210 suitable for use in the method of the present invention is described in EP 1 947 037 A1 by the same Applicant.

The speed V1 of the tape 1 and the pitch P1 at which the absorbent inserts 2 are positioned are directly proportional to each other. In fact, the speed V1 expressed in meters per second, is given by the pitch P1 expressed in meters multiplied by the number of absorbent articles 10 that the production line is able to produce in the unit of time, typically 1 second. In other words, the pitch P1 is equal to the transverse width 8 of the absorbent article 10 in an open and extended condition.

In the preferred embodiment, as illustrated in Figure 4, on the tape 1, between the first elastic structure 1a and the second elastic structure 1b, at the crotch region 3 on which the absorbent insert 2 is typically arranged, a series of cuts 6 of appropriate shape can be made, by means of a shaped cutting unit 60. Each of the cuts 62 removes a portion of material 65 from the elasticated tape 1, and defines the contour of the leg openings 11 of two consecutive sanitary articles 10 which, in the preferred embodiment illustrated in Figures 3a and 4, can be circumscribed by the elastic elements 17 for the legs.

The shaped cutting unit 60 in the production flow can be placed either downstream or upstream of the Turn and Repitch device 210. For example, contrary to what is shown in Figure 4, the shaped cutting unit 60 can be placed upstream of the Turn and Repitch unit 210 to avoid possible interference of the cutting blade of the unit 60 with the absorbent insert 2.

In the manufacturing method 40 of the absorbent sanitary article 10, according to the preferred embodiment illustrated in Figure 4, once the composite tape 100 is produced, consisting of a chain of semi-finished blanks of absorbent sanitary articles 10, the first elastic structure 1a and the second elastic structure 1b are overlapped on each other by folding the composite tape 100 longitudinally around a rod 36 of a longitudinal folding station 30. The bar 36 is arranged parallel to the direction of longitudinal advancing MD of the production line flow, and typically coinciding with the axis of symmetry X10 of the individual absorbent products 10, in order to bring together the longitudinal edges 9a and 9b of the composite tape 100.

In Figure 4, the arrow C schematically indicates the folding movement which achieves the overlapping of the first elastic structure 1a with the second elastic structure 1b and of the respective aforesaid longitudinal edges 9a and 9b.

An example of equipment and method 30 suitable for carrying out a fold of this type is described in the application TO2011A001085 by the same Applicant.

As illustrated in the preferred embodiment of Figure 4, in the device 40, the composite tape 100 passes through the longitudinal folding device 30 that carries out the overlapping of the first elastic structure 1a with the second elastic structure 1b, at the first speed V1. Homologous elements of consecutive absorbent sanitary articles 10, at this step of the process, are spaced apart by a first pitch P1.

Subsequently, after said overlapping operation of the first and second elastic structures 1a, 1b, the composite tape 100 is fed to a braking station 70 which reduces the speed by taking it from the first speed V1 to a second speed V2, typically between said first speed V1 of the tape 100 and the feeding speed V16 of the elastic elements 16. In this way, at the outlet of the braking station 70, the blanks of the consecutive absorbent sanitary articles 10, which make up the composite tape 100, slow down to said second speed V2 and are spaced apart by a second pitch P2.

The reduction in speed is made possible because the state of tension T1 of the composite tape 100 is partially released at the braking station 70 by bringing the elastic elements 16 closer to their rest condition. This involves the reduction of the distance between two consecutive products, which will have a second pitch P2 less than the first pitch P1 in the composite tape 100.

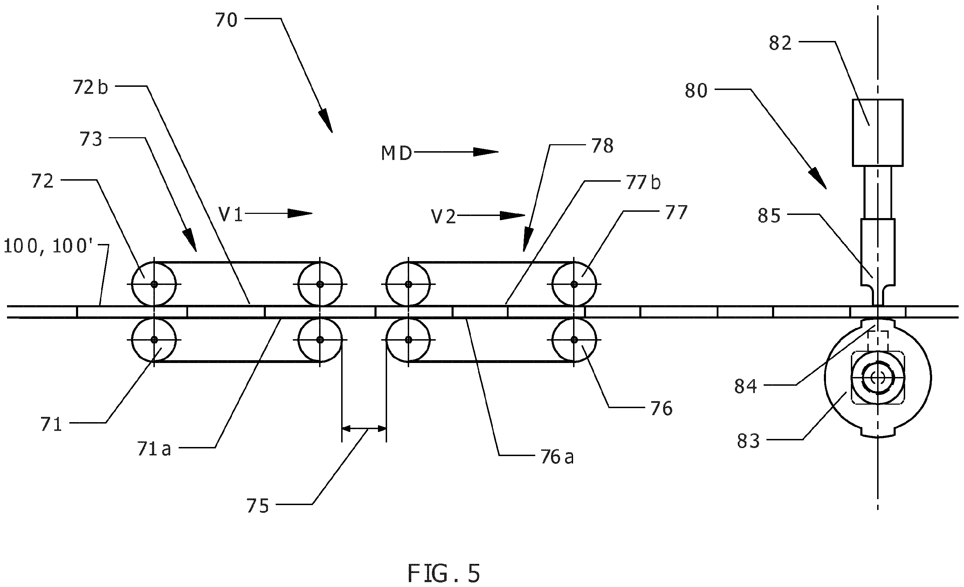

In the preferred embodiment illustrated in Figure 4, a particularly advantageous device 70 is represented to implement the braking of the tape 100, illustrated in greater detail in Figure 5.

With reference to Figure 5, the brake section 70 comprises a first pair of motorized conveyor belts 73, formed from a lower conveyor belt 71 and an upper conveyor belt 72, and a second pair of motorized conveyor belts 78, in turn formed from a lower conveyor belt 76 and an upper conveyor belt 77.

The lower conveyor belts 71 and 76 and the upper conveyor belts 72 and 77 of the conveyors 73 and 78 forming the braking device 70 are arranged, typically, on opposite sides of the tape 100 folded longitudinally. Therefore, each conveyor belt is in contact with a respective elastic structure 1a and 1b.

In a preferred embodiment, as is more evident in the view of Figure 5, in the braking device 70, the branches 71a, 72b and 76a, 77b of the conveyor belts 73 and 78, can be connected to a source of sub-atmospheric pressure in order to be able to carry out the gripping of the respective elastic structures 1a and 1b of the composite tape 100, for suction as well as pressure.

In the preferred embodiment, as illustrated in Figure 5, in the first conveyor belt 73, the branches 71a and 72b move in the direction MD at the first speed V1, while in the second conveyor belt 78, the branches 76a and 77b move in the direction MD at the second speed V2.

In the transition zone 75 between the first conveyor belt 73, which moves at the first speed V1 and the second conveyor belt 78, which moves at the second speed V2, the tape 100 is left free to release the tension T1 corresponding to the first speed V1, and slows down to the second speed V2, which corresponds to a second state of tension T2 less than T1.

The speed V2 of the composite tape 100, at the outlet of the braking station 70 is preferably greater than the speed V16 of the elastic elements 16 for the following reasons:
- to obtain a weld of acceptable quality, in effect, when the laminated tape 1 releases the tension T1, it will retract forming the characteristic "pleating" due to the fact that said elastic threads are integrally joined to the inner and outer sheets, creating, therefore, transverse wrinkles. The abovesaid pleating could prove detrimental to the process of downstream transverse welding - that will be described below - causing a non-uniform increase in thickness of material between the welding elements;
- to control the moving composite tape 100, it is necessary to maintain a minimum value of the longitudinal tension.

In the preferred embodiment of Figure 4, an advantageous speed V2 for the method of the present invention is in the range that can vary between a minimum value of 0.7 V1 and a maximum value of 0.9 V1.

After slowing down the composite tape 100 formed of the continuous chain of product blanks to the second speed V2, the latter is processed by the welding device 80 suitable for producing the transverse welds 13, which join together the first elastic structure 1a with the second elastic structure 1b, contributing to confer the characteristic pant-conformation to the absorbent article 10, defining the waist opening 14 and the two leg openings 11.

In the preferred embodiment illustrated in Figure 4 and in Figure 5, the welding operation is carried out by the equipment 80, which typically consists of an ultrasonic welding device 82 which cooperates with a counter-roller 83.

Typically, the tape 100 of blanks fed at the second speed V2 is passed through the welding apparatus 80 between the ultrasonic welding device 82 and the counter-roller 83.

The counter-roller 83 rotates in order to have the respective peripheral speed essentially identical to the advancing speed V2 of the composite tape 100 formed of the chain of blanks of sanitary articles 10.

An example of a method and equipment 80 suitable to carry out a transverse weld 13 of this type is described in WO 2010/101287 A1 by Yamamoto.

In the welding device described here, the vibrating apparatus of the ultrasonic system 82, which provides the energy to create the weld 13 of the first and second elastic structures 1a and 1b implements, as already said, a process of the forging type. In practice, in the apparatus 80, the welds 13 are produced by the vibrating device of the ultrasonic system 82, which repeatedly strikes, with a strong intensity, the layers of material interposed between it and the respective counter-roller 83, which is typically provided on its outer surface with appropriate welding blades 84 protruding transversely to the feed direction of the composite tape 100.

Due to the repeated blows, the material of the tape 100 interposed between the welding pattern and the vibrating element 85 of the ultrasonic system 82 is pressed, compressed, reduced in thickness and forced to migrate toward the side edges of the welding blades 84 of the counter-roller 83, generating the heat necessary to melt all the layers of material. An example of a counter-roller 83 provided with welding blades and suitable to carry out transverse welding lines 13 is described in TO2012A000210 by the same Applicant.

It is necessary to apply a minimum number of blows to the elastic structures 1a and 1b in order to obtain an acceptable weld, which typically varies according to the type of elastic structures 1a, 1b of the composite tape 100, the material, the number of layers and the configuration of the welding pattern.

The reduction of the crossing speed of the welding unit 80 by the composite tape 100 formed of the chain of blanks of sanitary articles 10 to the second speed V2, carried out by the braking device 70, allows the ultrasonic system, vibrating at a given frequency, to confer a greater number of blows to the composite tape 100 with respect to a line that processes a composite tape 100 at the first speed V1.

After making the transverse welds 13, the composite tape 100 is sent, at the second speed V2, to the cutting station 90, which segments it into a plurality of sanitary articles 10.

An example of a method and device 90 suitable to carry out the cutting operation in production lines of this type is described in TO2012A000209 by the same Applicant.

In the preferred embodiment, as shown in Figure 4, immediately after the longitudinal folding unit 30 and before the braking unit 70, it is possible to insert an additional welding device 180 that carries out technical welding, which can be, for example, welding points.

Said technical welds, which are designed to break easily at the time in which the absorbent sanitary article 10 is worn, have the function of maintaining the two elastic structures 1a and 1b integral with each other during the operations of deceleration of the composite tape 100 thus avoiding relative movements between the two elastic structures.

Devices suitable for carrying out technical welding for the aforesaid object are well known in the art, such as mechanical welding systems with heated rotating rollers, as represented in Figure 4, or ultrasonic welding devices. Examples of welding equipment 180 suitable for carrying out the technical welding as described above are described in US 4,919,738 A and US 4,854,984 A both by Ball et al.

The composite tape 100' of Figure 3b can be processed in the apparatus of Figure 4 in an analogous manner to that described for the composite tape 100 of Figure 3a, except for the fact that the two elastic structures 1a' and 1b' are produced independently from each other and joined by the inserts 2 so as to form a single tape 100'.

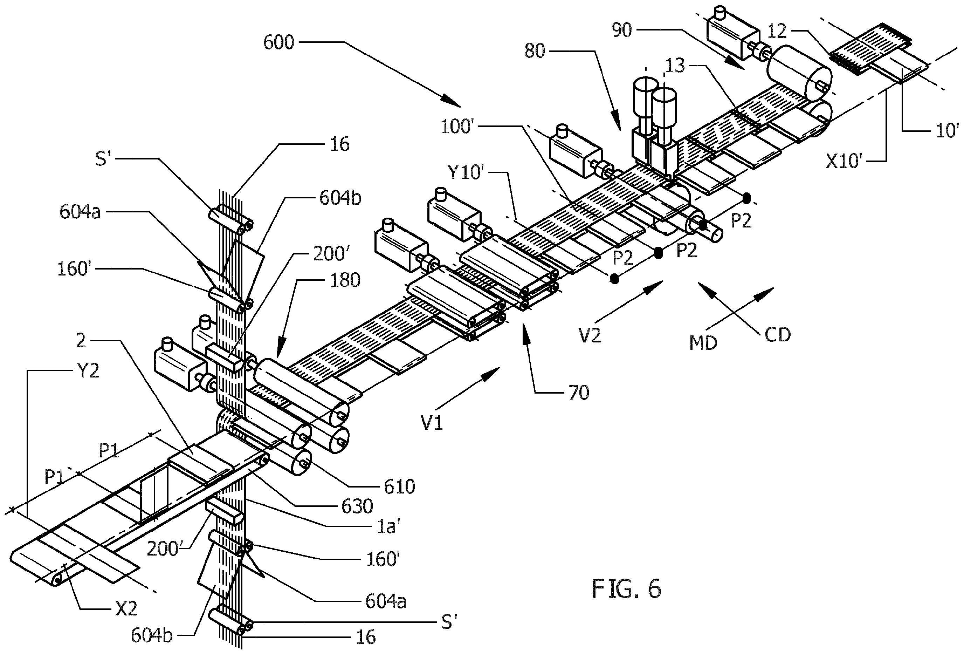

A further preferred embodiment of the present invention can be implemented, as illustrated in Figure 6, in the particularly advantageous method 600 for being able to produce an absorbent sanitary article 10', as schematically represented in Figure 3b, in which the first waist region 5 and the second waist region 15 are formed of two separate tapes parallel to each other and formed, respectively, by a first elastic structure 1a' and by a second elastic structure 1b', and where the absorbent insert 2, joining together the two elastic structures 1a' and 1b', contributes in forming the crotch region 3 and the leg openings 11.

For a better understanding, the elements and/or devices common to both the method 40 of Figure 4 and the method 600 of Figure 6 will be identified by the same numbers.

In the further preferred embodiment of Figure 6, the manufacturing method 600 of the absorbent sanitary article 10', includes a first step in which the two elastic structures 1a' and 1b' are produced. Each of the two elastic structures 1a' and 1b' can be produced by joining together a first sheet or inner sheet 604a, and a second sheet or outer sheet 604b with the elastic elements 16 interposed between said sheets.

The first and the second sheets 604a, 604b and the elastic elements 16, belonging to each of the two elastic structures 1a' and 1b', are joined together in respective fixing stations 160'.

As highlighted in Figure 6, the first elastic structure 1a' and the second elastic structure 1b' contribute to form, respectively, the first waist region 5 and the second waist region 15 of the absorbent sanitary article 10'.

In this case as well, the inner sheets 604a and the outer sheets 604b are supplied to the welding station 160' each from their own unwinding source, not illustrated in the figures but of a known type in the art.

Analogously, the elastic elements 16 conveyed to the respective fixing station 160' can come from a feed system S', also of known type.

In the embodiment illustrated in Figure 6, each of the inner sheets 604a and outer sheets 604b, can also be fed in this case to the respective fixing station 160' at a first speed V1.

The elastic threads 16, which form the first elastic structure 1a' and the second elastic structure 1b' that provide the elasticization of the first and the second waist regions 5, 15 of the absorbent sanitary article 10', are fed to the respective welding station 160' with a speed V16, less than said speed V1 of the respective sheets 604a, 604b.

In this case as well, the two speeds V1 and V16 can be very different from each other. Typically, the feeding speed V16 of the elastic elements 16 can be about half of the first feeding speed V1 of the respective sheets 604a, 604b. Said difference in speed is compensated, during the lamination step, by an elongation of the elastic threads 16 proportional to said difference in speed, in order to have elastic structures 1a' and 1b' with appropriate characteristics of elasticity and tension T1.

In the embodiment illustrated in Figure 6, the fixing unit 160', suitable for integrally and permanently combining the sheets 604a and 604b and the elastic threads 16, may be entirely similar to the fixing unit 160 described in the embodiment of Figure 4. Each of the fixing units 160' may be equipped with a pair of counter-rotating rollers that press against each other, in order to adhere together all materials that pass through it, and which contribute to form the respective elasticated structures 1a', 1b'. Typically, each of the said rollers may be provided with a non-stick treatment on their outer surface to reduce contamination by the adhesive, if used in the manufacturing process.

The absorbent inserts 2, in this further preferred embodiment illustrated in Figure 6, can also be produced according to a method known as "Machine Direction", so as to have a tape 201 of blanks of absorbent inserts 2 parallel to the MD direction of the production line flow. Typically, the tape 201 is segmented by a cutting unit (not shown in Figure 6), in order to produce a flow of individual absorbent inserts 2.

Each absorbent insert 2, analogously to that described in the previous embodiment of Figure 4, may be picked up by a Turn and Repitch device (not shown in Figure 6) that synchronizes the speed with the speed V1 of the elastic structures 1a' and 1b' and changes its orientation by rotating it by 90° in order to have the respective transversal axis X2 parallel to the production direction MD of the machine and coinciding with the axis X10' of the sanitary article 10', so as to create a plurality of absorbent inserts equidistant from each other and spaced at a constant pitch P1.

Unlike the embodiment of Figure 4, in the further preferred embodiment illustrated in Figure 6, the individual absorbent inserts 2 released from the Turn and Repitch device, instead of being deposited directly on the elastic structures 1a' and 1b', are released onto a longitudinal folding device 630 that folds each absorbent insert 2 in two, around the respective transverse axis X2.

An example of the method and the device 630 capable of carrying out the folding operation of the inserts 2 in production lines of this type is described in TO2010A000143 by the same Applicant.

In the embodiment of Figure 6, after having produced the first elastic structures 1a' and the second elastic structure 1b', these are sent to the additional fixing equipment 610 where the elastic structures 1a' and 1b' are joined to the absorbent inserts 2 already folded in two, typically by means of adhesive applied to each elastic structure 1a' and 1b' with respective application devices 200', which spread layers of adhesive of appropriate size and quantity at the position of the relative absorbent inserts 2, in order to produce a composite tape 100' formed of a continuous chain of blanks of sanitary articles 10'.

From this moment on, the composite tape 100' in the preferred embodiment of Figure 6 is treated in the same way in which the homologous composite tape 100 is treated in the method illustrated in Figure 4. Therefore, the composite tape 100' is firstly decelerated by the braking device 70 to the second speed V2, less than the first speed V1, but greater than the speed V16, and subsequently the welds 13 are made on the composite tape 100', which join together the first elastic structure 1a' and the second overlapping elastic structure 1b', contributing to confer the characteristic pant-conformation to the absorbent article 10', defining the waist opening 14 and the two leg openings 11.

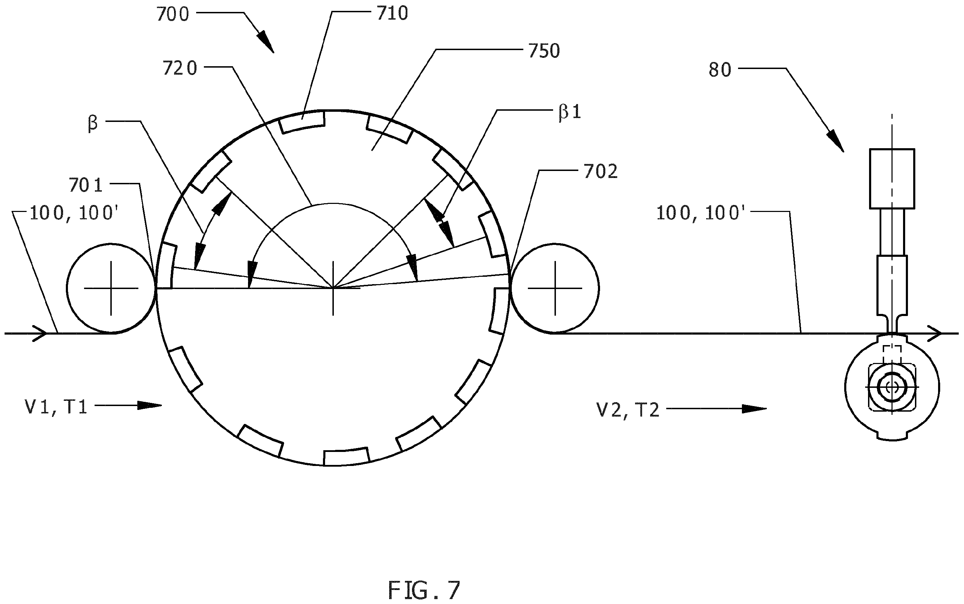

An alternative embodiment of a braking device 700 is illustrated in Figure 7. The composite tape 100, 100' at the output of the overlapping device 30, 610, and, preferably, after the welding unit 180, if present, which carries out the technical welding on the overlapping elastic structures 1a, 1a', 1b, 1b', is conveyed to a device for varying the pitch 750, which picks up the composite tape 100, 100' at the first speed V1 and at the respective first tension T1 at the pickup point 701 by means of suitable gripping elements 710 and returns it to the corresponding release point 702 at the second speed V2 and at the corresponding tension T2.

Each gripping element 710 grabs and holds the relative portion of the tape 100, 100' with which it comes into contact throughout the path 720 up to the release point 702 in which the tape 100, 100' is sent to the transverse welding unit 80. During the route between the pickup point and the release point, the gripping elements 710 vary their angular velocity, and consequently vary the peripheral speed of the relative gripping elements and of the tape held between them, as shown in Figure 7 in which, at the pickup point, consecutive gripping elements 710 subtend at an angle β greater than the corresponding angle β1 subtended by the same gripping elements when they are at the release point 702.

An example of a pitch variation device 750 suitable for use in the method of the present invention is described in EP 1 772 403 A1 by the same Applicant.

Of course, without prejudice to the principle of the invention, the details of construction and the embodiments may vary, even significantly, with respect to those illustrated here, purely by way of non-limiting example, without departing from the scope of the invention as defined by the attached claims. For example, the elastic elements 16 can be applied in a discontinuous manner, or the elastic structures 1a' and 1b' may be tapes of preformed elastic material and fed under tension by known means to the apparatus of the present invention.

## Claims

1. A method for the transverse welding of a composite tape (100, 100') comprising the steps of:
- forming a composite tape (100, 100') comprising a first and a second elastic structure (1a, 1a', 1b, 1b') formed in a fixing station (160) and overlapping each other, which is continuously advanced in a longitudinal direction (MD) at a first feeding speed (V1) and at a first feed tension (T1), wherein each of said elastic structures (1a, 1a', 1b, 1b') comprises a first web material (4a) and a second web material (4b) with interposed elastic elements (16) fed to said fixing station (160) with a feeding speed (V16), less than said first feeding speed (V1);
- slowing down (70) said composite tape (100, 100'), taking it to a second speed (V2) less than said first feeding speed (V1) and greater than said feeding speed (V16) of said elastic elements (16), and a second tension (T2) less than said first feed tension (T1),
- feeding said composite tape (100, 100') at said second speed (V2) and tension (T2) to a welding station (80) and welding (80) said composite tape (100, 100') with welding lines (13) transverse to said longitudinal feed direction (MD), and
- feeding said composite tape (100, 100') at said second speed (V2) and tension (T2) to a cutting station (90) and cutting (90) said composite tape (100, 100') with cuts (12) transverse to said longitudinal feed direction (MD),
wherein said slowing down step of said composite tape (100, 100') is carried out by feeding said composite tape (100, 100') at said first speed (V1) to a braking device (700) which releases said composite tape (100, 100') at said second speed (V2).

2. A method according to claim 1, wherein said slowing down step of said composite tape (100, 100') comprises the step of picking up the composite tape (100, 100') at the first speed (V1) and at the first tension (T1) at a pickup point (701) by means of gripping elements (710) and returning said composite tape (100, 100') to a release point (702) at the second speed (V2) and at the second tension (T2), wherein said braking device (700) comprises a device for varying the pitch (750) provided of gripping elements (710).

3. A method according to any one of the preceding claims, wherein during the route between the pickup point (701) and the release point (702), said gripping elements (710) vary their angular velocity and peripheral speed for varying the peripheral speed of the composite tape (100, 100') held between them so that, at the pickup point (701), consecutive gripping elements (710) subtend an angle (β) greater than the corresponding angle (β1) subtended by the same gripping elements (710) when they are at the release point (702) .

4. A method according to any one of the preceding claims, wherein said feeding speed (V16) of said elastic elements (16) is about half of the first feeding speed (V1) of said sheets (4a, 4b).

5. A method according to any one of the preceding claims, wherein the two elastic structures (1a, 1b) are longitudinal end portions of a single tape (1, 100), wherein said elastic structures (1a, 1b) overlap each other by means of folding (30) said single tape (100) along its longitudinal axis (X10).

6. A method according to any one of claims from 1 to 4, wherein the two elastic structures (1a', 1b') are transversely joined by discrete elements (2) equidistant (P1') to each other and folded around a transverse axis (X2), wherein the method comprises the step of forming a composite tape structure (100'), wherein said elastic structures (1a', 1b') are applied one of top of the other with portions of said discrete elements (2) arranged between said folded elastic structures (1a', 1b').

7. A method according to any one of the preceding claims, comprising a technical welding step (180) of said overlapping (30, 610) elastic structures (1a, 1a', 1b, 1b') before said slowing down step (70).

8. Apparatus for the transverse welding of a composite tape (100, 100') comprising at least two overlapping elastic structures (1a, 1a', 1b, 1b'), which is fed to the apparatus continuously in a longitudinal direction (MD) at a first speed (V1) and at a first tension (T1), said apparatus comprising:
- a fixing station (160) adapted to form said elastic structures (1a, 1a', 1b, 1b') each comprising a first web material (4a) and a second web material (4b) with interposed elastic elements (16), wherein said elastic elements (16) are fed to said fixing station (160) with a feeding speed (V16), less than said first feeding speed (V1);
- a welding device (80) adapted to produce welding lines (13) transverse to said longitudinal feed direction (MD) on said composite tape (100, 100') traveling at a second speed (V2) less than said first feeding speed (V1) and greater than said feeding speed (V16) of said elastic elements (16), and a second tension (T2) less than said first feed tension (T1);
- a braking device (700) adapted to be fed with said composite tape (100, 100') at said first speed (V1) and to release said composite tape (100, 100') at the second speed (V2) to the welding device (80), wherein said braking device (700) is configured to slow down said composite tape (100, 100') from the first speed (V1) to the second speed (V2).

9. Apparatus according to claim 8, wherein said braking device (700) comprises a device for varying the pitch (750) provided of gripping elements (710) adapted to pick up the composite tape (100, 100') at the first speed (V1) and at the first tension (T1) at a pickup point (701) and to return said composite tape (100, 100') to a release point (702) at the second speed (V2) and at the second tension (T2).

10. Apparatus according to claim 8 or 9, wherein said gripping elements (710) are configured to grab and hold relative portion of said composite tape (100, 100'), and to vary their angular velocity and peripheral speed so that, at the pickup point (701), consecutive gripping elements (710) subtend an angle (β) greater than the corresponding angle (β1) subtended by the same gripping elements (710) at the release point (702).

11. Apparatus according to any one of the claims from 8 to 10, wherein said feeding speed (V16) of said elastic elements (16) is about half of the first feeding speed (V1) of said sheets (4a, 4b).

12. Apparatus according to any one of claims from 8 to 11, comprising a welding device (180) adapted to form technical welds on said first and second overlapping elastic structures (1a, 1a', 1b, 1b') on said composite tape (100, 100') traveling at said first speed (V1), said welding device (180) being located upstream of said braking device (70).
